(19) Europäisches Patentamt
European Patent Office
Office européen des brevets

(11) **EP 4 678 681 A1**

(12) **EUROPÄISCHE PATENTANMELDUNG**

(43) Veröffentlichungstag:
**14.01.2026 Patentblatt 2026/03**

(21) Anmeldenummer: **24188320.6**

(22) Anmeldetag: **12.07.2024**

(51) Internationale Patentklassifikation (IPC):
***C08G 65/40*** (2006.01) ***B01J 41/13*** (2017.01)
***B32B 27/00*** (2006.01) ***C07D 211/00*** (2006.01)
***C08G 75/23*** (2006.01) ***C08J 5/00*** (2006.01)
***C08L 71/00*** (2006.01) ***C08L 81/06*** (2006.01)
***C09D 181/06*** (2006.01) ***C09J 181/06*** (2006.01)
***C25B 13/00*** (2006.01) ***H01M 8/00*** (2016.01)

(52) Gemeinsame Patentklassifikation (CPC):
**C08G 65/40; B01J 41/13; B32B 27/00; C07D 211/00; C08G 65/4056; C08G 75/23; C08J 5/00; C08L 71/00; C08L 81/06; C09D 181/06; C09J 181/06; C25B 1/04; C25B 13/08**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(71) Anmelder: **Evonik Operations GmbH**
**45128 Essen (DE)**

(72) Erfinder:
- **HARTMANN, Sven Sören**
  **45657 Recklinghausen (DE)**
- **SCHESTAKOV, Maria**
  **46282 Dorsten (DE)**

(74) Vertreter: **Evonik Patent Association**
**c/o Evonik Industries AG**
**IP Management**
**Postcode 84/339**
**Rodenbacher Chaussee 4**
**63457 Hanau (DE)**

(54) **HERSTELLEN VON POLYMEREN FÜR DIE AEM-WASSERELEKTROLYSE MIT VERRINGERTER QUELLUNGSNEIGUNG**

(57) Die Erfindung befasst sich mit der Herstellung von Anionen leitenden Polymeren, welche für den Einsatz in der alkalischen Membranwasserelektrolyse bestimmt sind. Konkret soll die Quellungsneigung von Anionen leitenden Polymeren in alkalischen wässrigen Lösungen reduziert werden, ohne dass die spezifische Anionenleitfähigkeit dieser Polymere stark beeinträchtigt wird. Dies gelingt durch die Zugabe eines weiteren Edukts in das Reaktionsgemisch. Insgesamt wird das Polymer aus drei Edukten hergestellt, nämlich aus 4,4-bis-(4-hydroxy-3,5-dimethyl-phenyl)-1-methyl-piperidin (Formel I), aus 4,4'-Difluorbenzophenon (Formel II), sowie aus 4,4'-Dihydroxybenzophenon (Formel III) bzw. aus Bis(4-hydroxy-3,5-dimethylphenyl)methanone (Formel IV).

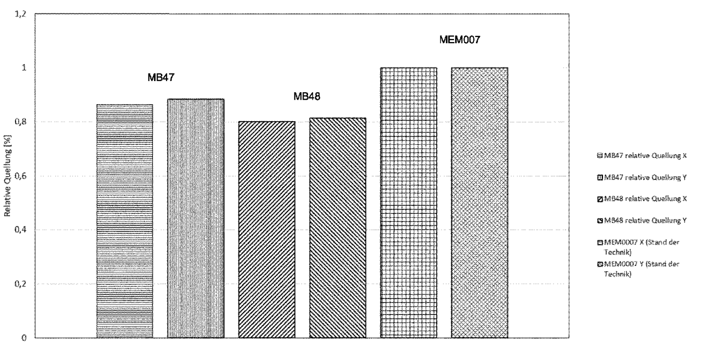


Fig. 1



Processed by Luminess, 75001 PARIS (FR)

## Beschreibung

Die Erfindung befasst sich mit der Herstellung von Anionen leitenden Polymeren, welche für den Einsatz in der alkalischen Membranwasserelektrolyse bestimmt sind.

Die Wasserelektrolyse beschreibt die Zerlegung von Wasser $H_2O$ in Wasserstoff $H_2$ und Sauerstoff $O_2$ durch elektrischen Strom. Dabei kann die Wasserelektrolyse sowohl im alkalischen Medium als auch im sauren Medium durchgeführt werden. Die alkalische Wasserelektrolyse nutzt einen basischen Elektrolyten, die saure einen sauren. Der Elektrolyt dient dem Austausch der Ionen und enthält zugleich das zu spaltende Wasser. Die Wasserelektrolyse wird mit einer elektrochemischen Zelle durchgeführt.

Die alkalische Membranwasserelektrolyse (engl: anion exchange membrane water electrolysis-AEMWE) ist eine besondere Verfahrensweise einer Wasserelektrolyse. Sie findet in basischen Milieu statt und wird in Gegenwart einer Anionenaustauschmembran durchgeführt. Die AEMWE kommt als eine Technologie zur nachhaltigen Produktion von "grünem" Wasserstoff in Betracht, sofern die erforderliche elektrische Energie regenerativ gewonnen wird. Vor diesem Hintergrund wird derzeit an der technischen Umsetzung der alkalische Membranwasserelektrolyse im industriellen Maßstab gearbeitet.

Einen Überblick über den Aufbau der gegenwärtig in der AEMWE eingesetzten elektrochemischen Zellen und der darin verwendeten Materialien bieten:

Miller, Hamish Andrew et al: Green hydrogen from anion exchange membrane water electrolysis: a review of recent developments in critical materials and operating conditions. Sustainable Energy Fuels, 2020, 4, 2114 DOI: 10.1039/c9se01240k

Insbesondere für die Herstellung der in der AEMWE eingesetzten Membranen werden Polymere mit Leitfähigkeit für Anionen, genauer gesagt, für Hydroxid-Ionen (OH-) benötigt.

Polymere mit Leitfähigkeit für Anionen sind unter anderem aus WO 2021/013694 A1, EP4032934A1 und EP4059988A1 bekannt. Sie eignen sich zur Herstellung von Anionenaustauschmembranen (engl. anion exchange membranes - AEM). Diese Membranen können wiederum in der alkalischen Wasserselektrolyse eingesetzt werden.

Eine wesentliche Eigenschaft, welche ein Polymer für seine Eignung als Membranmaterial in der AEMWE qualifiziert, ist zunächst seine hohe spezifische Leitfähigkeit für Hydroxid-Ionen ($OH^-$).

Im technischen Kontext ist es darüber hinaus von besonderem Interesse, dass das Polymer in einer wässrigen alkalischen Lösung möglichst nicht quillt: Unter Quellen ist die Zunahme des Volumens eines aus dem Polymer hergestellten Bauteils der elektrochemischen Zelle zu verstehen, welches auf das Eindringen der alkalischen Lösung in das Polymer zurückzuführen ist. Mit alkalischer Lösung kommt die Membran in der AEMWE prinzipbedingt dauerhaft in Kontakt, weil die wässrige alkalische Lösung (meist Kalilauge KOH) als Elektrolyt genutzt wird.

Die durch die Quellung des Polymers hervorgerufene Volumenzunahme geht in der Regel mit einer anisotropen Gestaltänderung des aus dem Polymer hergestellten Bauteils der elektrochemischen Zelle einher. Im Falle der Membran führt diese Gestaltänderung zu Undichtigkeiten an der Zelle, was im Interesse der Betriebssicherheit der Anlage tunlichst zu vermeiden ist: Bei der Wasserelektrolyse entstehen gasförmiger Wasserstoff und Sauerstoff, die durch die Membran getrennt gehalten werden. Wenn die Membran aufgrund des Quellens undicht wird, vermischen sich Wasserstoff und Sauerstoff und bilden hochexplosives Knallgas.

Eine tiefer gehende Beschreibung des Quellvorgangs von Anionen leitenden Polymeren findet sich in:

Qiongjuan Duan, Shanhai Ge, Chao-Yang Wang: Water uptake, ionic conductivity and swelling properties of anion-exchange membrane, Journal of Power Sources, Volume 243, 2013, Pages 773-778, ISSN 0378-7753, https://doi.org/10.1016/j.jpowsour.2013.06.095.

Die Gruppe um Qiongjuan Duan hat sich mit dem Quellen von Anionenaustauschmembranen befasst, welche innerhalb von Brennstoffzellen stattfindet. In Brennstoffzellen erfolgt keine Wasserelektrolyse, sondern eine Synthese von Wasser unter Abgabe von elektrischer Energie. Da hier keine alkalischen Bedingungen herrschen, wurde das Quellen der Polymere durch neutrales Wasser untersucht.

Neben der physikalischen Quellung werden die in der AEMWE eingesetzten Anionen leitenden Polymere auch durch die Base chemisch angegriffen. Das Polymer wird dadurch über einen längeren Zeitraum degradiert.

Die in WO 2021/013694 A1 und EP4032934A1 offenbarten, Anionen leitenden Polymere wurden in diesen Schriften lediglich hinsichtlich ihrer Quellstabilität in deionisierten Wasser untersucht. Eine Untersuchung hinsichtlich der Degradation der Polymere durch basische Belastung erfolgte indes nicht. Insoweit ist keine Aussage über die Langzeitstabilität dieser Polymere im basischen Elektrolyten möglich.

In der EP4059988A1 wurde die Langzeitstabilität von diversen Anionen leitenden Polymeren in Kalilauge bei 80°C untersucht. Dabei wurde auch ein aus WO 2021/013694 A1 bekanntes Polymer verglichen, siehe Membran #1 aus Example 27.

Es soll an dieser Stelle nicht unerwähnt bleiben, dass neben des Quellens im Elektrolyse-Betrieb auch schon während der Herstellung der Anionenaustauschmembran das Quellen der dafür verwendeten Anionen leitenden Polymere Schwierigkeiten bereitet: So werden AEM gerne mit Katalysatoren bzw. katalytisch aktiven Materialien

beschichtet, um die Effizienz der Elektrolyse zu steigern. Die dabei erzeugten Katalysatorschichten enthalten neben dem katalytisch aktiven Material auch Bindemittel, welches das katalytisch aktive Material auf der Membran immobilisieren. Als Bindemittel werden ebenfalls Anionen leitende Polymere verwendet. Wenn beim Auftragen der Katalysatorschicht das Anionen leitende Polymer quillt, führt dies zu einer fehlerhaften Beschichtung, welche den Einbau der beschichten Membran in die Zelle stark erschwert. In diesem Zusammenhang ist allerdings zu bemerken, dass das Quellen des Ionen leitenden Polymers während der Produktion der Anionenaustauschmembran in der Regel von in der Membranproduktion eingesetzten, organischen Lösemitteln verursacht wird. Im Betrieb der AEMWE sind diese Lösemittel in der Regel nicht mehr relevant.

Aus der Sicht der Hersteller und Anwender von für die alkalische Wasserelektrolyse bestimmten Anionenaustauschmembranen ist es also bedeutsam, dass die Anionen leitenden Polymere, aus denen die AEM hergestellt werden, weder in den produktionsbedingt genutzten organischen Lösemitteln, noch in den als Elektrolyt genutzten alkalischen wässrigen Lösungen quellen.

Diese Erfindung befasst sich schwerpunktmäßig mit solchen Maßnahmen, die das Quellen während der Elektrolyse betreffen. Konkret soll die Quellungsneigung von Anionen leitenden Polymeren in alkalischen wässrigen Lösungen reduziert werden, ohne dass die spezifische Anionenleitfähigkeit dieser Polymere stark beeinträchtigt wird.

Gelöst wird diese Aufgabe dadurch, dass die Polymere wie folgt hergestellt werden:

a) Bereitstellen eines Reaktionsgemisches enthaltend:

- ein erstes Edukt, nämlich 4,4-bis-(4-hydroxy-3,5-dimethyl-phenyl)-1-methylpiperidin;
- ein zweites Edukt, nämlich 4,4'-Difluorbenzophenon;
- ein drittes Edukt, bei dem es sich um 4,4'-Dihydroxybenzophenon und/oder um Bis(4-hydroxy-3,5-dimethylphenyl)methanone handelt;
- mindestens ein erstes Lösemittel;
- mindestens ein Deprotonierungsmittel;

b) Beaufschlagen des Reaktionsgemisches mit Wärme, sodass das Reaktionsgemisch eine Temperatur zwischen 140°C und 180°C annimmt;
c) Abscheiden von Wasser aus dem Reaktionsgemisch;
d) Erhalt eines Vorprodukts aus dem Reaktionsgemisch;
e) Waschen des Vorprodukts;
f) Trocknen des gewaschenen Vorprodukts;
g) Bereitstellen einer Alkylierungsreagenz;
h) Kontaktieren der Alkylierungsreagenz mit dem gewaschenen und getrockneten Vorprodukt;
i) Erhalt des Polymers.

Ein solches Verfahren ist ein erster Gegenstand der Erfindung.

Die Erfindung beruht auf der unerwarteten Erkenntnis, dass durch die Zugabe eines weiteren Edukts in das Reaktionsgemisch ein Polymer erhalten wird, welches eine erhöhte Quellbeständigkeit in Lauge aufweist, verglichen zu einem Polymer, welches ohne dieses zusätzliche Edukt hergestellt wurde.

Überraschend ist außerdem, dass die Anionenleitfähigkeit des durch das zusätzliche Edukt erhaltenen Polymers kaum niedriger ist als die eines herkömmlichen Polymers, welches - von dem zusätzlichen Edukt abgesehen - auf denselben Ausgangsmaterialien basiert.

Konkret wird das Polymer aus drei Edukten hergestellt. Das erste Edukt ist 4,4-bis-(4-hydroxy-3,5-dimethylphenyl)-1-methyl-piperidin, welches in der Formel I dargestellt ist.

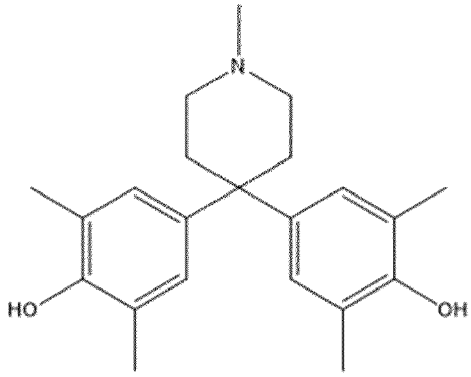

(I)

Die in diesem Baustein enthaltene Piperidin-Gruppe wird durch Quaternisierung positiv geladen und bedingt so die intrinsische Anionenleitfähigkeit des Polymers.

Bei dem zweiten Edukt handelt es sich um 4,4'-Difluorbenzophenon gemäß Formel II:

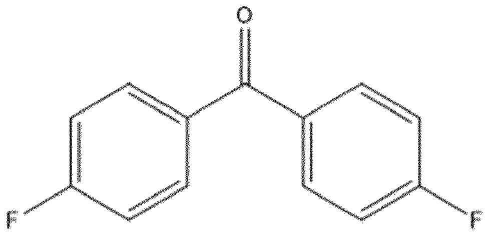

(II)

Dieser Baustein dient der äquimolare Einwaage von Hydroxidverbindung (Bausteine I und III bzw. IV) und Fluorverbindung. Demnach wird die Einwaage vom Baustein II in Abhängigkeit der Einwaage von Bausteinen I und III/IV gewählt.

Die Nutzung der Bausteine (I) und (II) ist aus der WO 2021/013694 A1 bekannt.

Erfindungsgemäß wird noch ein drittes Edukt im Reaktionsgemisch vorgelegt, welches wahlweise in methylierter Form oder unmethyliert eingesetzt werden kann.

In einfacher, unmethylierter Form handelt es sich bei dem dritten Edukt um 4,4'-Dihydroxybenzophenon, welches in Formel (III) dargestellt ist.

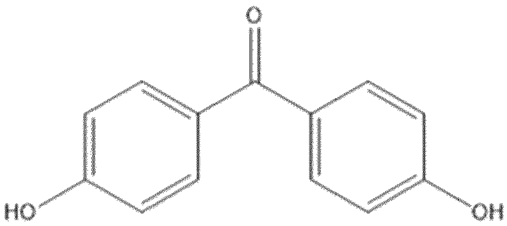

(III)

Alternativ kann als drittes Edukt das in Formel (IV) dargestellte Bis(4-hydroxy-3,5-dimethylphenyl)methanone verwendet werden.

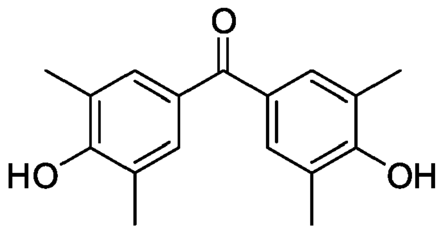

(IV)

Der in Formel (IV) dargestellte Baustein ist die methylierte Form von (III). Selbstverständlich kann als drittes Edukt auch ein Gemisch aus Baustein (III) und Baustein (IV) verwendet werden.

Abgesehen von der Auswahl der im bereitgestellten Reaktionsgemisch enthaltenen Edukte, entspricht das erfindungsgemäße Herstellverfahren der herkömmlichen Herstellung von Anionen leitenden Polymeren mit Piperidin-Gruppe, in Wege der Polykondensation und anschließender Quaternisierung.

Wie bei jeder chemischen Reaktion sollten auch hier die drei Edukte in einem geeigneten Stoffmengenverhältnis zueinander bereitgestellt werden. In einer besonders bevorzugten Ausführungsform der Erfindung werden die Stoffmengen *u*, *v*, *w* so gewählt, dass die Beziehungen gemäß Formel (V) und (VI) eingehalten werden:

$$u = v \cdot \frac{100-c}{100} \qquad \text{(V)}$$

$$w = v \cdot \frac{c}{100} \qquad \text{(VI)}$$

In den Beziehungen gemäß Formel (V) und (VI) steht jeweils u für die Stoffmenge des ersten Edukts, *v*, für die Stoffmenge des zweiten Edukts und *w* für die Stoffmenge des dritten Edukts. Sofern ein Gemisch der Bausteine (III) und (IV) als drittes Edukt verwendet wird, beschreibt w die Stoffmenge des Gemisches der Bausteine (III) und (IV). Die Gesamte Stoffmenge, also die Größe des Ansatzes, wird über die Stoffmenge w des dritten Edukts skaliert. Theoretisch kann auch über alle Bausteine skaliert werden, da die Stoffmengen über die Formen (V) und (VI) miteinander zusammen hängen.

Der Formelbuchstabe c steht in beiden Formel (V) und (VI) jeweils eine rationale Zahl zwischen 0 und 15. Wichtig ist, dass *c* ist in beiden Formeln dieselbe Zahl repräsentiert.

Die Zahl c definiert einen Ersetzungsfaktor. Dies bedeutet, dass zwischen 0 und 15 % der Stoffmenge des ersten Edukts *u* durch die Stoffmenge des dritten Edukts w ersetzt ist. Die Zahl *c* kann beispielsweise 5 oder 10 betragen. Aus der Addition der Formeln (V) und (VI) folgt, dass die Summe der Stoffmengen u und *w* der Stoffmenge *v* entspricht. Das erste Edukt und das dritte Edukt nehmen somit gemeinsam denselben stöchiometrischen Anteil am Reaktionsgemisch ein wie das zweite Edukt.

Die hier angegebenen Stoffmengen *u, v, w* beschreiben die optimale Stöchiometrie, die einer äquimolaren Einwaage entspricht. Freilich ist es möglich, von der optimalen Stöchiometrie abzuweichen und einzelne Edukte tatsächlich im Unterschuss oder Überschuss zuzugeben. Das ist aber nicht bevorzugt, weil dies zu kürzeren Polymerketten führt: Die Molmasse der erhaltenen Polymere ist dann zu niedrig und deren Viskosität damit auch. Die Membran wird im Ergebnis zu weich und lässt sich nicht so gut assemblieren. Außerdem wird die Membran anfälliger gegenüber mechanischer Degradation, bei welcher das Backbone des Polymers gespalten wird. Wenn die Polymerkette von Anfang an kürzer ist, kommt es schneller zur Unterschreitung einer kritischen Kettenlänge, bei der die Membran versprödet. Wenn die Kettenlänge bereits zu Beginn infolge einer von der Formel (V) bzw. (VI) nicht unwesentlich abweichenden Einwaage die kritische Kettenlänge unterschreitet, besteht das Risiko, dass sich aus der erhaltenen Polymerlösung keine Membran mehr gießen lässt. Insbesondere wird keine homogene Beschichtung des beim Membrangießen verwendeten Substrats mehr erhalten. Aus diesen Gründen werden die Edukte vorzugsweise tatsächlich in dem optimalen stöchiometrischen Verhältnis im Reaktionsgemisch bereitgestellt.

Das Deprotonierungsmittel wird in der Reaktion teilweise verbraucht, aber nicht in das Polymer eingebaut. Vorzugsweise handelt es sich bei dem Deprotonierungsmittel um ein Alkalicarbonat, ganz besonders bevorzugt um Lithiumcarbonat oder um Natriumcarbonat oder Kaliumcarbonat oder um Mischungen daraus.

Nach Abschluss der Polymerisation der drei Edukte wird ein Vorprodukt erhalten. Das Vorprodukt ist zu großen Teilen noch im ersten Lösemittel gelöst, insbesondere bei Reaktionstemperatur. Das Vorprodukt kann aus dem Reaktionsgemisch gewonnen werden, in dem es in destilliertes Wasser abgelassen wird. Um es von dem Lösemittel, nicht umgesetzten Edukt und nicht umgesetzten Deprotonierungsmittel zu reinigen, wird das Vorprodukt mit Wasser gewaschen und anschließend getrocknet, sodass das Waschwasser wieder entfernt wird. Das Waschen und Trocknen lässt sich am einfachsten durchführen, wenn das Vorprodukt im rohen Zustand, im gewaschenen Zustand oder spätestens

während des Trocknens mit Scherung beaufschlagt wird. Dabei wird das Vorprodukt zerkleinert. Das Scheren des Vorprodukts kann mit einem Mischer oder einem Mixer oder einem Kneter erfolgen.

Das Vorprodukt ist noch nicht leitfähig für Anionen. Hierfür muss es quaternisiert werden. Dies geschieht vorzugweise dadurch, dass das Vorprodukt nach dem Waschen und Trocken zumindest teilweise in einem zweiten Lösemittel aufgelöst und in Gegenwart des zweiten Lösemittels mit dem Alkylierungsreagenz kontaktiert wird. Vorzugweise erfolgt die Quaternisierung bei leicht erhöhter Temperatur, etwa im Bereich von 30°C bis 60°C.

Als Alkylierungsreagenz wird vorzugweise ein Halogenalkan wie beispielsweise Iodmethan oder Brommethan verwendet. Mischungen davon sind auch als Alkylierungsreagenz einsetzbar.

Das Verfahren erfordert bis zu zwei Lösemittel, nämlich ein erstes, welches in dem Reaktionsgemisch enthalten ist und in welchem die Polymerisation durchgeführt wird sowie ein zweites, in welchem die Quaternisierung erfolgt. Die beiden Lösemittel können gleich oder unterschiedlich sein. Vorzugsweise handelt es sich bei dem ersten und/oder dem zweiten Lösemittel um eine Substanz, die aus der Gruppe bestehend aus N,N-Dimethylacetamid (DMAC), N,N-Dimethylformamid (DMF), N-Methyl-2-pyrrolidon (NMP), Dimethylsulfoxid (DMSO), Acetonitril (ACN), Ethanol (EtOH), Methanol (MeOH) ausgewählt ist. Selbstverständlich können auch Mischungen dieser Substanzen als Lösemittel eingesetzt werden. Besonders bevorzugt werden DMAC oder NMP als erstes Lösemittel genutzt und DMSO, ACN oder EtOH als zweites Lösemittel.

Da das Anionen leitende Polymer erfindungsgemäß aus drei Edukten hergestellt wird, handelt es sich bei dem Produkt um ein Terpolymer. Aufgrund der Ähnlichkeit von den Bausteinen (III) und (IV) wird ein Polymer, welches aus allen vier Bausteinen I, II, III, IV hergestellt wird, ebenfalls als Terpolymer aufgefasst.

Der strukturelle Aufbau des Terpolymers lässt sich nicht hinreichend genau beschrieben. Dies liegt daran, da es sich bei der Synthese um eine Polykondensationsreaktion handelt. Das 4,4'-Difluorbenzophenon (II) fungiert dabei als Elektrophil, mit Fluor als Abgangsgruppe. Als Nucleophil kann entweder das erste Edukt (I) oder das dritte Edukt (III) und/oder (IV) fungieren. Der tatsächliche Aufbau der Polymerkette ist daher statistisch verteilt. Hinzu kommt, dass die Kettenlänge und die Länge einer einzigen Wiederholeinheit des Terpolymers von der gewählten Einsatzmenge der Edukte abhängt.

Eine theoretisch mögliche Struktur des erhaltenen Terpolymers ist in Formel VII dargestellt:

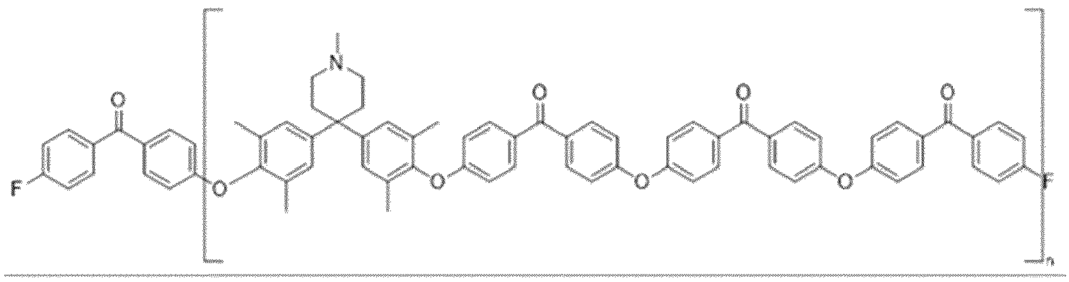

(VII)

Da sowohl die Anzahl der Wiederholeinheiten n, als auch die Anzahl und Abfolge der Bausteine innerhalb der Wiederholeinheit stark variieren kann, wird das tatsächlich erhaltene Produkt eine Vielfalt unterschiedlicher Terpolymere enthalten.

Aufgrund der kaum darstellbaren Komplexität des Produktes ist ein Polymer, welches durch das erfindungsgemäße Verfahren erhältlich ist, ein weiterer Gegenstand der Erfindung.

Das erhaltene Polymer ist nach der Quaternisierung Anionen leitfähig und hinreichend beständig in alkalischer Lösung. Es lässt sich daher in der alkalischen Membranwasserelektrolyse verwenden. Diese Verwendung ist ein weiterer Gegenstand der Erfindung.

Die Verwendung erfolgt dadurch, dass eine alkalische Membranwasserelektrolyse in Gegenwart des hergestellten Polymers durchgeführt wird. Ein Verfahren zur Herstellung von Wasserstoff und Sauerstoff durch Elektrolyse von Wasser im basischen Milieu, durchgeführt in Anwesenheit des Polymers, ist daher ein weiterer Gegenstand der Erfindung.

Konkret lässt sich aus dem Polymer eine Anionenaustauschmembran (AEM) herstellen. Das Polymer bildet dabei den Anionen leitenden Membranwerkstoff. Das Polymer kann mit anderen Materialien vermischt werden. Eine Anionenaustauschmembran, enthaltend ein erfindungsgemäß erhaltenes Polymer ist daher ein weiterer Gegenstand der Erfindung.

Ein weiterer Gegenstand der Erfindung ist eine Zusammensetzung, welche mindestens ein drittes Lösemittel und ein darin zumindest teilweise gelöstes, erfindungsgemäß erhaltenes Polymer enthält.

Eine solche Zusammensetzung ist viskos und dient dazu, eine Anionenaustauschmembran aus dem Polymer herzustellen. Das erfolgt dadurch, dass die Zusammensetzung auf ein Substrat aufgetragen und getrocknet wird. Beim Trocknen verflüchtigt sich das dritte Lösemittel, sodass das filmbildende Polymer ausfällt und eine Schicht auf dem

Substrat bildet. Als Substrat kann beispielsweise eine Glasplatte verwendet werden. Von der Glasplatte abgelöst wird die Schicht als AEM genutzt. Ein solcher Vorgang ist als "membrane casting" bekannt.

Das in der Zusammensetzung verwendete dritte Lösemittel kann dasselbe sein, wie das im Reaktionsgemisch enthaltene erste Lösemittel und/oder das in der Quaternisierung verwendete zweite Lösemittel. Das dritte Lösemittel kann aber auch davon unterschiedlich sein.

Im Übrigen kann die Zusammensetzung zusätzlich mindestens einen partikulären Elektrokatalysator enthalten. Elektrokatalysatoren beschleunigen elektrochemische Reaktionen, ohne dabei selbst verbraucht zu werden. Eine Zusammensetzung enthaltend einen Elektrokatalysator wird abhängig von ihrer Viskosität als "Katalysatortinte" oder als "Katalysatorpaste" bezeichnet.

Die Katalysatortinte/paste wird ebenfalls auf ein Substrat aufgetragen und getrocknet, dass sich eine feste Katalysatorschicht auf dem Substrat ausbildet. Die Katalysatorschicht wird von dem aus der Lösung ausgefallenen Anionen leitenden Polymer und dem darin dispergierten, partikulären Elektrokatalysator gebildet. Als Substrat dient einfachstenfalls eine Anionenaustauschmembran, sodass diese eine katalytisch aktive Schicht erhält. Der Elektrokatalysator ist über das als Binder dienende Anionen leitende Polymer auf der AEM immobilisiert. Ein solch erhaltener Schichtkörper wird auch als "catalytic coated membrane" (CCM) bezeichnet. Ein Verfahren zur Herstellung einer CCM ist in WO 2023/088714 A1 offenbart. Dabei wird aus einem Ionomer und einem Elektrokatalysator eine Katalysatortinte hergestellt, diese auf eine AEM direkt aufgesprüht und getrocknet. Das hier beschriebene Anionen leitende Polymer kann analog verarbeitet werden.

## Beispiele

Die Erfindung soll nun anhand von Synthesebeispielen näher erläutert werden. Sodann werden die beispielhaft hergestellten Polymere hinsichtlich ihrer Anionenleitfähigkeit, Ionenaustauschkapazität und Quellungsneigung untersucht. Es zeigt:

Figur 1: relative Quellung (Dimensionsänderung) der erfindungsgemäßen Membranen gegenüber der herkömmlichen Membran, jeweils in X- und Y-Richtung.

1. Herstellung einer AEM aus herkömmlichen Polymer (nicht Teil der Erfindung)

Ein Anionen leitendes Polymer wurde entsprechend der Beispiele 1 bis 3 der WO 2021/013694 A1 synthetisiert. Aus dem Polymer wurde entsprechend Beispiel 4 der genannten WO-Schrift eine Anionenaustauschmembran gegossen. Die Probenbezeichnung war MEM007.

2.1 Herstellung des Vorprodukts mit *c*=5 eines erfindungsgemäßem Polymers (Teil der Erfindung)

Die Synthese wurde in einem 2 L Doppelwand Glasreaktor, mit einer Blatt-Rührwelle, einem Wasserabscheider und unter Stickstoff-Gegenstrom durchgeführt. Zur Beginn der Synthese wurden 0,667 mol (226,77 g) von 4,4-bis-(4-hydroxy-3,5-dimethyl-phenyl)-1-methyl-piperidin, 0,035 mol (7,52 g) von Dihydroxybenzophenon, 0,702 mol (153,27 g) von Difluorbenzophenon, 1,544 mol (213,58 g) von Kaliumcarbonat und 1250 mL Dimethylacetamid vorgelegt und bei 90 Umdrehungen pro Minute unter Stickstoff-Gegenstrom für 30 Minuten bei Raumtemperatur gerührt. Anschließend wurde die Temperatur des Thermostaten auf 165 °C erhöht. Die Temperatur wurde für 19 h bei 165 °C Manteltemperatur gehalten. Die Innentemperatur des Reaktionsgemisches betrug 160 °C. Das entstandene Wasser wurde über den kompletten Zeitraum, über den Wasserabscheider, unter Stickstoffstrom, aus dem System getragen. Anschließend wurde die heiße Reaktionsmasse aus dem Reaktor in Reinstwasser (~20 °C) mit turbulenter Strömung, unter starker Scherung, portionsweise abgelassen. In Summe wurden die ca. 1400 mL Reaktionsgemisch in ca. 6000 mL Wasser abgelassen. Das Material wurde anschließend sechsmal mit jeweils 2000 mL Reinstwasser (60 °C) gewaschen. Das gewaschene, weiße Polymermaterial, wurde anschließend bei 80 °C im Vakuumtrockenschrank für 72 h bei 200 mbara (800 mbar Unterdruck) getrocknet. Die Auswaage betrug 87 %.

2.2 Herstellung des Vorprodukts mit *c*=10 eines erfindungsgemäßen Polymers (Teil der Erfindung)

Die Synthese wurde in einem 2 L Doppelwand Glasreaktor, mit einer Blatt-Rührwelle, einem Wasserabscheider und unter Stickstoff-Gegenstrom durchgeführt. Zur Beginn der Synthese wurden 0,632 mol (226,77 g) von 4,4-bis-(4-hydroxy-3,5-dimethyl-phenyl)-1-methyl-piperidin, 0,070 mol (15,04 g) von Dihydroxybenzophenon, 0,702 mol (153,27 g) von Difluorbenzophenon, 1,544 mol (213,58 g) von Kaliumcarbonat und 1250 mL Dimethylacetamid vorgelegt und bei 90 Umdrehungen pro Minute unter Stickstoff-Gegenstrom für 30 Minuten bei Raumtemperatur gerührt. Die Synthese wurde analog zu der unter 2.1 Beschriebenen durchgeführt. Die Auswaage betrug 82 %.

3.1 Quaternisierung des Vorprodukts mit c=5 (Teil der Erfindung)

Das in 2.1 erhaltene Vorprodukt wurde analog zu Beispiel 3 der WO 2021/013694 A1 quaternisiert. Dadurch wurde ein Anionen leitendes Polymer erhalten.

3.2 Quaternisierung des Vorprodukts mit *c*=10 (Teil der Erfindung)

Das in 2.2 erhaltene Vorprodukt wurde analog zu Beispiel 3 der WO 2021/013694 A1 quaternisiert. Dadurch wurde ein Anionen leitendes Polymer erhalten.

4.1 Herstellung einer AEM aus erfindungsgemäßen Polymer mit c=5 (Teil der Erfindung)

Die Polymerlösung, welche aus 3.1 erhalten wurde, wurde durch eine Filtrationsapparatur mit 1 μm PTFE Filtergewebe geleitet. Anschließend wurde die Polymerlösung mittels Rakel auf eine PET-Folie aufgetragen. Der Rakel bewegte sich dabei gleichbleibend mit 5 mm/s. Die PET-Folie befand sich während des Prozesses auf einer 70 °C warmen Heizbank. Zum Trocknen verblieb die PET-Folie zusammen mit dem aufgetragenen Polymerfilm 1 h auf der Heizbank. Die Probenbezeichnung war MB47.

4.2 Herstellung einer AEM aus erfindungsgemäßen Polymer mit c=10 (Teil der Erfindung)

Die Polymerlösung, welche aus 3.2 erhalten wurde, wurde durch eine Filtrationsapparatur mit 1 μm PTFE Filtergewebe geleitet. Anschließend wurde die Polymerlösung mittels Rakel auf eine PET-Folie aufgetragen. Der Rakel bewegte sich dabei gleichbleibend mit 5 mm/s. Die PET-Folie befand sich während des Prozesses auf einer 70 °C warmen Heizbank. Zum Trocknen verblieb die PET-Folie zusammen mit dem aufgetragenen Polymerfilm 1 h auf der Heizbank. Die Probenbezeichnung war MB48.

5. Bestimmung des Quellverhaltens

Neben der Veränderung der Polarität und sich daraus ergebene Veränderungen im Löslichkeitsverhalten und der darauffolgenden Prozessierung, ist insbesondere die Quellung eine wichtige Einflussgröße, die durch die strukturelle Änderung (5 mol% bzw. 10 mol% Difluorbenzophenon im erfindungsgemäßen Polymer) beeinflusst wird.

Aus den unter 1. und 4.1 bzw. 4.2 hergestellten AEM wurden jeweils mittels Stanzeisen drei Membranstücke (flache Gestalt, Maß 25 mm × 15 mm) vorbereitet.

Die Membranstücke wurden allesamt bei 50 °C für 24 h bei Atmosphärendruck getrocknet und deren Größe anschließend mittels Lichtmikroskop vermessen. Anschließend wurden die Membranstücke in 1 M KOH-Lösung (60 °C) für 24 h, im Schüttelwasserbad gelagert. Danach wurden die Membranstücke zwei Maljeweils für 30 Minuten bei 60 °C in Reinstwasser, im Schüttelwasserbad gelagert. Dann wurde das Reinstwasser noch einmal mit frischem Wasser ausgetauscht und bei 20°C bis 25 °C innerhalb der nächsten 30 Minuten vermessen. Die Größen der Membranstücke wurden erneut mittels Lichtmikroskop vermessen. Die dimensionale Änderung ergibt sich aus der Differenz der aufgenommenen Daten zwischen dem jeweiligen nassen Membranstück und dem getrockneten Membranstück.

Figur 1 zeigt die aus jeweils drei Einzelmessungen gemittelten Daten der relativen Quellung (Dimensionsänderung) des erfindungsgemäß hergestellten Materials gegenüber des herkömmlich hergestellten Materials.

Das erfindungsgemäß hergestellte Materials MB0047 (c=5) ist gegenüber des herkömmlich hergestellten Materials MEM007 in X-Richtung um 13,6% weniger gequollen, in Y-Richtung um 11,7%. Für MB48 (c = 10) ist der Effekt noch einmal verstärkt: Die Quellung verringert sich in X-Richtung um 20,0% und in Y-Richtung um 18,5%.

6. Bestimmung der Ionenaustauschkapazität für MEM007 und MB47

Die Ionenaustauschkapazität (IEC) ist eine Größe zur Bestimmung des Quaternisierungsgrades des Materials. Anders ausgedrückt, mittels IEC wird die Anzahl der geladenen Gruppen innerhalb des Polymers bestimmt. Das Vergleichsmaterial aus WO 2021/013694 A1 liefert einen theoretischen IEC-Wert von 1,681 mmol/g. Die gemessenen IEC-Werte liegen üblicherweise bei einem Quaternisierungsgrad von 99 - 100 (+/- 1) %. Bei dem erfindungsgemäß hergestellten Material MB47 konnte ein Quaternisierungsgrad von 97,5 (+/- 0,6) % gemessen werden. Die leichte Abnahme in der IEC kann auf eine Verringerung der funktionellen Gruppen durch das Einbauen des Bausteins gemäß Formel (III) in das Polymergerüst zurückzuführen sein.

7. Bestimmung der Anionenleitfähigkeit für MB47

Die Leitfähigkeit (LF) der Membran für Hydroxidionen ($OH^-$) ist ein weiterer Indikator dafür, wie effizient die Membran in der Elektrolyse performt. Eine geringe Ionenleitfähigkeit führt zu einem höheren Widerstand in der Zelle, was zu Spannungsverlusten führt, welche wiederrum die Effizienz der Elektrolyse durch den anfallenden Wärmeverlust verringern.

Die LF und IEC korrelieren miteinander, da die LF bedeutend von der Anzahl der geladenen Gruppen im Polymer abhängt. Allerdings unterliegen die LF-Messungen höheren Schwankungen als die IEC-Messungen.

Die LF wird nicht direkt gemessen, sondern der Flächenwiderstand der Oberfläche.

Für die Bestimmung werden drei Membranstücke aus der Membran MB47 ausgestanzt und anschließend in Hydroxidform Ionengetauscht. Die Membran wird mittels 4-Punkt-Methode vermessen.

Die erhaltenen LF-Werte des erfindungsmäßigen Materials entsprechen -95% des herkömmlichen Materials (Cyclovoltammetrie CV). Die Abnahme in der Leitfähigkeit ist durch die Abnahme der geladenen Gruppen zu begründen. Diese ist durch den Ersatz von 5% der Verbindung, welche den quaternären Stickstoff trägt, gemäß Formel (I), durch den Baustein gemäß Formel (III) und/oder (IV) bedingt.

8. Fazit

Durch die Verwendung des zusätzlichen Bausteins III und durch die 5 %-ige bzw. 10 %-ige Substitution des Bausteins (I) durch Baustein (III) (und/oder IV) wird ein Polymer erhalten, dessen Quellverhalten in KOH gegenüber einem solchen Material reduziert ist, welches ausschließlich aus (I) und (II) im stöchiometrischen Verhältnis 1:1 hergestellt wurde.

Allerdings werden durch diese Maßnahmen die geladenen Gruppen in der Membran reduziert, was sich durch eine Abnahme ihrer Ionenleitfähigkeit bemerkbar macht. Da aber durch eine verringerte Quellung neue Applikationen erschlossen werden können (z.B. Einbau größerer Membranen in Elektrolyseure, bessere Beschichtung mit Katalysatoren aufgrund einer besseren Haftung) ist dieser Nachteil tolerabel.

## Patentansprüche

**1.** Verfahren zur Herstellung eines Polymers mit den folgenden Schritten:

a) Bereitstellen eines Reaktionsgemisches enthaltend:

- ein erstes Edukt, nämlich 4,4-bis-(4-hydroxy-3,5-dimethyl-phenyl)-1-methylpiperidin (Formel I);
- ein zweites Edukt, nämlich 4,4'-Difluorbenzophenon (Formel II);
- mindestens ein erstes Lösemittel;
- mindestens ein Deprotonierungsmittel;

b) Beaufschlagen des Reaktionsgemisches mit Wärme, sodass das Reaktionsgemisch eine Temperatur zwischen 140°C und 180°C annimmt;
c) Abscheiden von Wasser aus dem Reaktionsgemisch;
d) Erhalt eines Vorprodukts aus dem Reaktionsgemisch;
e) Waschen des Vorprodukts;
f) Trocknen des gewaschenen Vorprodukts;
g) Bereitstellen einer Alkylierungsreagenz;
h) Kontaktieren der Alkylierungsreagenz mit dem gewaschenen und getrockneten Vorprodukt;
i) Erhalt des Polymers;

**dadurch gekennzeichnet,**

**dass** das Reaktionsgemisch zusätzlich ein drittes Edukt enthält,
wobei es sich bei dem dritten Edukt um 4,4'-Dihydroxybenzophenon (Formel III) und/oder um Bis(4-hydroxy-3,5-dimethylphenyl)methanone (Formel IV) handelt.

**2.** Verfahren nach Anspruch 1, wobei das Reaktionsgemisch so bereitgestellt wird,

dass die drei Edukte jeweils mindestens in einer Stoffmenge *u*, *v*, *w* vorliegen,

wobei *u* für die Stoffmenge des ersten Edukts, *v* für die Stoffmenge des zweiten Edukts und *w* für die Stoffmenge des dritten Edukts steht, **dadurch gekennzeichnet,**
**dass** für *u*, *v*, *w* gilt:

$$u = v \cdot \frac{100-c}{100} \qquad (V)$$

$$w = v \cdot \frac{c}{100} \qquad (VI)$$

worin *c* eine rationale Zahl zwischen 0 und 15 ist, welche in Formel (V) und Formel (VI) gleich gewählt ist.

**3.** Verfahren nach Anspruch 1 oder 2, **dadurch gekennzeichnet, dass** das Vorprodukt und/oder das gewaschene Vorprodukt und/oder das gewaschene und getrocknete Vorprodukt mit Scherung beaufschlagt wird.

**4.** Verfahren nach einem der Ansprüche 1 bis 3, **dadurch gekennzeichnet, dass** das gewaschene und getrocknete Vorprodukt in Gegenwart eines zweiten Lösemittels mit der Alkylierungsreagenz kontaktiert wird, wobei das gewaschene und getrocknete Vorprodukt zumindest teilweise in dem zweiten Lösemittel gelöst ist.

**5.** Verfahren nach einem der Ansprüche 1 bis 4, **dadurch gekennzeichnet, dass** es sich bei dem Deprotonierungsmittel um ein Alkalicarbonat handelt.

**6.** Verfahren nach Anspruch 5, **dadurch gekennzeichnet, dass** das Deprotonierungsmittel ausgewählt ist aus der Gruppe bestehend aus: Lithiumcarbonat, Natriumcarbonat, Kaliumcarbonat.

**7.** Verfahren nach einem der Ansprüche 1 bis 6, **dadurch gekennzeichnet, dass** es sich bei dem Alkylierungsreagenz um ein Halogenalkan handelt.

**8.** Verfahren nach einem der vorhergehenden Ansprüchen 1 bis 7, **dadurch gekennzeichnet, dass** das erste Lösemittel und/oder das zweite Lösemittel ausgewählt ist aus der Gruppe bestehend aus N,N-Dimethylacetamid (DMAC), N,N-Dimethylformamid (DMF), N-Methyl-2-pyrrolidon (NMP), Dimethylsulfoxid (DMSO), Acetonitril (ACN), Ethanol (EtOH), Methanol (MeOH).

**9.** Polymer, erhältlich durch ein Verfahren nach einem der Ansprüche 1 bis 8.

**10.** Verwendung des Polymers nach Anspruch 9 in der alkalischen Membranwasserelektrolyse.

**11.** Verfahren zur Herstellung von Wasserstoff und Sauerstoff durch Elektrolyse von Wasser im basischen Milieu, durchgeführt in Anwesenheit eines Polymers nach Anspruch 9.

**12.** Anionenaustauschmembran enthaltend ein Polymer nach Anspruch 9.

**13.** Zusammensetzung mindestens enthaltend die folgenden Komponenten:

i) ein drittes Lösemittel;
ii) ein Polymer nach Anspruch 9, welches in dem Lösemittel zumindest teilweise gelöst ist.

**14.** Zusammensetzung nach Anspruch 13, zusätzlich enthaltend
iii) mindestens ein partikulärer Elektrokatalysator.

**15.** Verfahren zum Herstellen einer Anionenaustauschmembran nach Anspruch 12, bei dem eine Zusammensetzung nach Anspruch 13 und/oder nach Anspruch 14 bereitgestellt, auf ein Substrat aufgetragen und getrocknet wird.

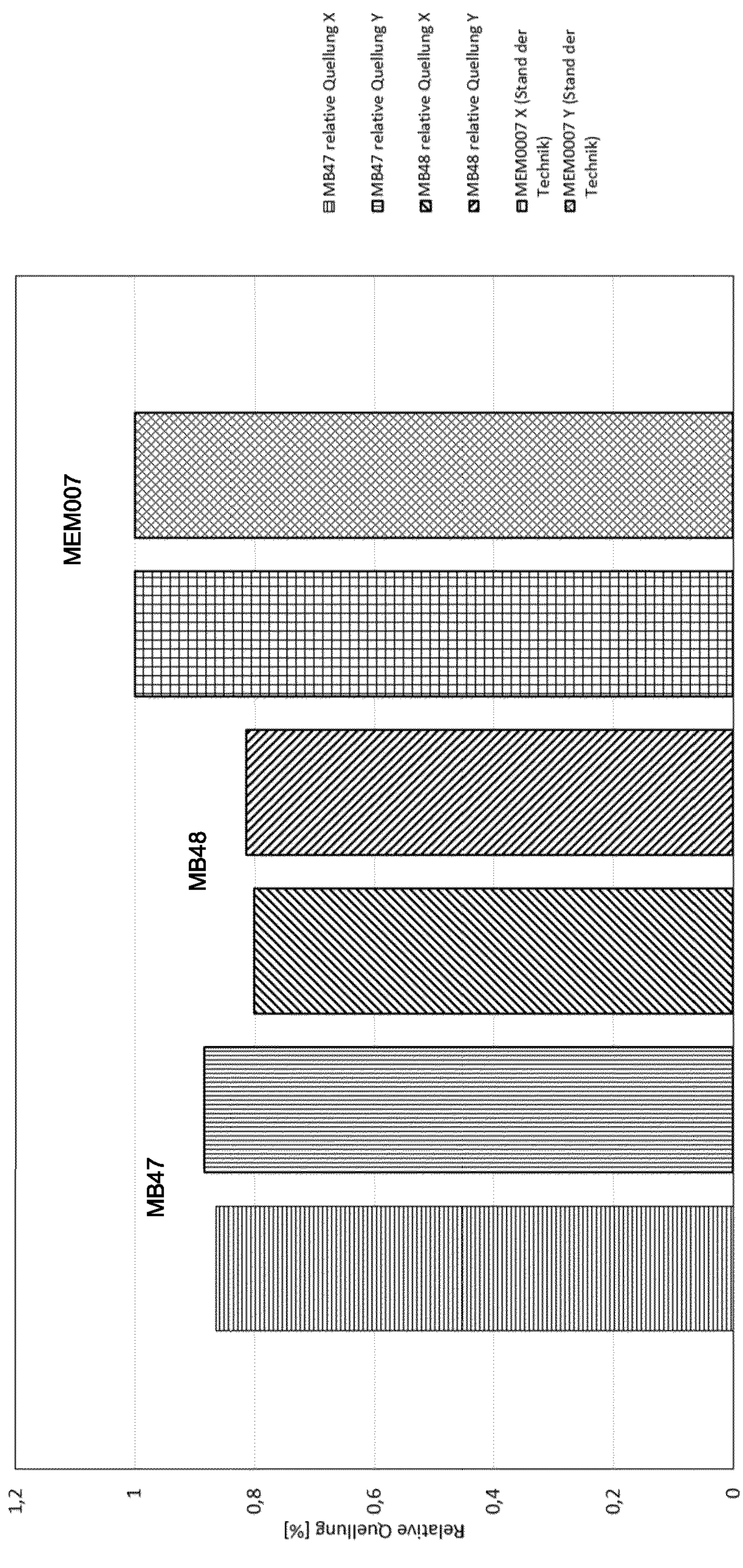
MB47 relative Quellung X
MB47 relative Quellung Y
MB48 relative Quellung X
MB48 relative Quellung Y
MEM0007 X (Stand der Technik)
MEM0007 Y (Stand der Technik)
MEM007
MB48
MB47
1,2
1
0,8
0,6
0,4
0,2
0
Relative Quellung [%]

Fig. 1

Europäisches Patentamt
European Patent Office
Office européen des brevets

# EUROPÄISCHER RECHERCHENBERICHT

Nummer der Anmeldung
EP 24 18 8320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | CN 113 563 576 A (CHANGCHUN INST APPLIED CHEMISTRY CAS) 29. Oktober 2021 (2021-10-29) * Absatz [0001] - Absatz [0025]; Ansprüche 1-6; Beispiele * | 1-15 | INV. C08G65/40 B01J41/13 B32B27/00 C07D211/00 C08G75/23 C08J5/00 C08L71/00 C08L81/06 C09D181/06 C09J181/06 C25B13/00 H01M8/00 |
| A | EP 3 770 201 A1 (EVONIK OPERATIONS GMBH [DE]) 27. Januar 2021 (2021-01-27) * Absatz [0001] - Absatz [0030]; Ansprüche 1-13; Beispiele * | 1-15 | |
| A | WO 2021/013694 A1 (EVONIK OPERATIONS GMBH [DE]) 28. Januar 2021 (2021-01-28) * Seite 1, Zeile 2 - Seite 8, Zeile 5; Ansprüche 1-13; Beispiele * | 1-15 | |
| A | CN 110 294 845 A (CHANGCHUN INST APPLIED CHEMISTRY CAS) 1. Oktober 2019 (2019-10-01) * Absatz [0001] - Absatz [0028]; Ansprüche 1-10; Beispiele * | 1-15 | RECHERCHIERTE SACHGEBIETE (IPC) |
| A | CN 106 750 303 A (UNIV JILIN) 31. Mai 2017 (2017-05-31) * Absatz [0001] - Absatz [0030]; Ansprüche 1-10; Beispiele * | 1-15 | C08G C25B H01M C08J B01J B32B C07D C08L C09D C09J |
| | -/-- | | |

Der vorliegende Recherchenbericht wurde für alle Patentansprüche erstellt

1

| Recherchenort | Abschlußdatum der Recherche | Prüfer |
|---|---|---|
| Den Haag | 12. Dezember 2024 | Kiebooms, Rafaël |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Seite 1 von 3

Europäisches Patentamt
European Patent Office
Office européen des brevets

# EUROPÄISCHER RECHERCHENBERICHT

Nummer der Anmeldung
EP 24 18 8320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | LI SU ET AL: "Anion conductive piperidinium based poly (ether sulfone): Synthesis, properties and cell performance", JOURNAL OF MEMBRANE SCIENCE, ELSEVIER BV, NL, Bd. 594, 13. September 2019 (2019-09-13), XP085877527, ISSN: 0376-7388, DOI: 10.1016/J.MEMSCI.2019.117471 [gefunden am 2019-09-13] * Seite 117471 - Seite 117477 * ----- | 1-15 | |
| A | WANG FEN ET AL: "Synthesis and property of novel anion exchange membrane based on poly(aryl ether sulfone)s bearing piperidinium moieties", JOURNAL OF MEMBRANE SCIENCE, ELSEVIER BV, NL, Bd. 591, 1. August 2019 (2019-08-01), XP085764659, ISSN: 0376-7388, DOI: 10.1016/J.MEMSCI.2019.117334 [gefunden am 2019-08-01] * Seite 117334 - Seite 117342 * ----- | 1-15 | RECHERCHIERTE SACHGEBIETE (IPC) |
| A,D | WO 2023/088714 A1 (EVONIK OPERATIONS GMBH [DE]) 25. Mai 2023 (2023-05-25) * Seite 1, Zeile 4 - Seite 13, Zeile 8; Ansprüche 1-13; Beispiele * ----- | 1-15 | |
| A,D | EP 4 032 934 A1 (EVONIK OPERATIONS GMBH [DE]) 27. Juli 2022 (2022-07-27) * Absatz [0001] - Absatz [0043]; Ansprüche 1-7; Beispiele * ----- -/-- | 1-15 | |

Der vorliegende Recherchenbericht wurde für alle Patentansprüche erstellt

1

| Recherchenort | Abschlußdatum der Recherche | Prüfer |
|---|---|---|
| Den Haag | 12. Dezember 2024 | Kiebooms, Rafaël |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Seite 2 von 3

Europäisches Patentamt
European Patent Office
Office européen des brevets

# EUROPÄISCHER RECHERCHENBERICHT

Nummer der Anmeldung
EP 24 18 8320

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A,D | EP 4 059 988 A1 (EVONIK OPERATIONS GMBH [DE]) 21. September 2022 (2022-09-21)<br>* Absatz [0001] - Absatz [0048]; Ansprüche 1-17; Beispiele *<br>----- | 1-15 | |
| A,D | MILLER HAMISH ANDREW ET AL: "Green hydrogen from anion exchange membrane water electrolysis: a review of recent developments in critical materials and operating conditions",<br>SUSTAINABLE ENERGY & FUELS,<br>Bd. 4, Nr. 5, 6. Mai 2020 (2020-05-06), Seiten 2114-2133, XP093103727,<br>DOI: 10.1039/C9SE01240K<br>* Seite 2114 - Seite 2133 *<br>----- | 1-15 | RECHERCHIERTE SACHGEBIETE (IPC) |

Der vorliegende Recherchenbericht wurde für alle Patentansprüche erstellt

1

| Recherchenort | Abschlußdatum der Recherche | Prüfer |
|---|---|---|
| Den Haag | 12. Dezember 2024 | Kiebooms, Rafaël |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 24 18 8320

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-12-2024

| Im Recherchenbericht angeführtes Patentdokument | Datum der Veröffentlichung | Mitglied(er) der Patentfamilie | Datum der Veröffentlichung |
|---|---|---|---|
| CN 113563576 A | 29-10-2021 | KEINE | |
| EP 3770201 A1 | 27-01-2021 | AU 2020317550 A1 | 10-03-2022 |
| | | BR 112022000236 A2 | 22-02-2022 |
| | | CA 3144717 A1 | 28-01-2021 |
| | | CL 2022000121 A1 | 11-11-2022 |
| | | CN 114144453 A | 04-03-2022 |
| | | DK 3770201 T3 | 21-08-2023 |
| | | EP 3770201 A1 | 27-01-2021 |
| | | ES 2953064 T3 | 07-11-2023 |
| | | FI 3770201 T3 | 14-08-2023 |
| | | HR P20230889 T1 | 10-11-2023 |
| | | HU E062445 T2 | 28-11-2023 |
| | | IL 289865 A | 01-03-2022 |
| | | JP 7503126 B2 | 19-06-2024 |
| | | JP 2022541056 A | 21-09-2022 |
| | | KR 20220038676 A | 29-03-2022 |
| | | LT 3770201 T | 25-08-2023 |
| | | MA 55236 A1 | 30-06-2022 |
| | | PL 3770201 T3 | 25-09-2023 |
| | | PT 3770201 T | 18-08-2023 |
| | | RS 64435 B1 | 29-09-2023 |
| | | SI 3770201 T1 | 29-09-2023 |
| | | TN 2021000238 A1 | 04-07-2023 |
| | | TW 202106755 A | 16-02-2021 |
| | | UA 128747 C2 | 09-10-2024 |
| | | US 2022243012 A1 | 04-08-2022 |
| | | WO 2021013694 A1 | 28-01-2021 |
| | | ZA 202201985 B | 29-03-2023 |
| WO 2021013694 A1 | 28-01-2021 | AU 2020317550 A1 | 10-03-2022 |
| | | BR 112022000236 A2 | 22-02-2022 |
| | | CA 3144717 A1 | 28-01-2021 |
| | | CL 2022000121 A1 | 11-11-2022 |
| | | CN 114144453 A | 04-03-2022 |
| | | DK 3770201 T3 | 21-08-2023 |
| | | EP 3770201 A1 | 27-01-2021 |
| | | ES 2953064 T3 | 07-11-2023 |
| | | FI 3770201 T3 | 14-08-2023 |
| | | HR P20230889 T1 | 10-11-2023 |
| | | HU E062445 T2 | 28-11-2023 |
| | | IL 289865 A | 01-03-2022 |
| | | JP 7503126 B2 | 19-06-2024 |
| | | JP 2022541056 A | 21-09-2022 |
| | | KR 20220038676 A | 29-03-2022 |
| | | LT 3770201 T | 25-08-2023 |

EPO FORM P0461

Für nähere Einzelheiten zu diesem Anhang : siehe Amtsblatt des Europäischen Patentamts, Nr.12/82

Seite 1 von 3

## ANHANG ZUM EUROPÄISCHEN RECHERCHENBERICHT ÜBER DIE EUROPÄISCHE PATENTANMELDUNG NR.

EP 24 18 8320

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-12-2024

| Im Recherchenbericht angeführtes Patentdokument | Datum der Veröffentlichung | Mitglied(er) der Patentfamilie | Datum der Veröffentlichung |
|---|---|---|---|
| | | MA 55236 A1 | 30-06-2022 |
| | | PL 3770201 T3 | 25-09-2023 |
| | | PT 3770201 T | 18-08-2023 |
| | | RS 64435 B1 | 29-09-2023 |
| | | SI 3770201 T1 | 29-09-2023 |
| | | TN 2021000238 A1 | 04-07-2023 |
| | | TW 202106755 A | 16-02-2021 |
| | | UA 128747 C2 | 09-10-2024 |
| | | US 2022243012 A1 | 04-08-2022 |
| | | WO 2021013694 A1 | 28-01-2021 |
| | | ZA 202201985 B | 29-03-2023 |
| CN 110294845 A | 01-10-2019 | KEINE | |
| CN 106750303 A | 31-05-2017 | KEINE | |
| WO 2023088714 A1 | 25-05-2023 | AR 127685 A1 | 21-02-2024 |
| | | AU 2022391917 A1 | 27-06-2024 |
| | | CA 3238362 A1 | 25-05-2023 |
| | | CN 118266105 A | 28-06-2024 |
| | | EP 4181240 A1 | 17-05-2023 |
| | | EP 4434101 A1 | 25-09-2024 |
| | | KR 20240113766 A | 23-07-2024 |
| | | TW 202335356 A | 01-09-2023 |
| | | WO 2023088714 A1 | 25-05-2023 |
| EP 4032934 A1 | 27-07-2022 | AR 124654 A1 | 19-04-2023 |
| | | AU 2022211566 A1 | 07-09-2023 |
| | | CA 3205168 A1 | 28-07-2022 |
| | | CN 116745341 A | 12-09-2023 |
| | | EP 4032934 A1 | 27-07-2022 |
| | | JP 2024503388 A | 25-01-2024 |
| | | KR 20230131215 A | 12-09-2023 |
| | | TW 202237693 A | 01-10-2022 |
| | | US 2024301153 A1 | 12-09-2024 |
| | | WO 2022157019 A1 | 28-07-2022 |
| EP 4059988 A1 | 21-09-2022 | AR 125534 A1 | 26-07-2023 |
| | | AU 2022236195 A1 | 02-11-2023 |
| | | CA 3212249 A1 | 22-09-2022 |
| | | CN 116997591 A | 03-11-2023 |
| | | EP 4059988 A1 | 21-09-2022 |
| | | JP 2024511718 A | 15-03-2024 |
| | | KR 20230156915 A | 15-11-2023 |
| | | TW 202302213 A | 16-01-2023 |
| | | US 2024166811 A1 | 23-05-2024 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 24 18 8320

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-12-2024

| Im Recherchenbericht angeführtes Patentdokument | Datum der Veröffentlichung | Mitglied(er) der Patentfamilie | Datum der Veröffentlichung |
|---|---|---|---|
| | | WO 2022194605 A1 | 22-09-2022 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2021013694 A1
- EP 4032934 A1
- EP 4059988 A1
- WO 2023088714 A1


### In der Beschreibung aufgeführte Nicht-Patentliteratur


- **MILLER, HAMISH ANDREW et al.** Green hydrogen from anion exchange membrane water electrolysis: a review of recent developments in critical materials and operating conditions. *Sustainable Energy Fuels*, 2020, vol. 4, 2114
- **QIONGJUAN DUAN** ; **SHANHAI GE** ; **CHAO-YANG WANG**. Water uptake, ionic conductivity and swelling properties of anion-exchange membrane. *Journal of Power Sources*, 2013, vol. 243, ISSN 0378-7753, 773-778, https://doi.org/10.1016/j.jpowsour.2013.06.095